# EUROPEAN PATENT APPLICATION

(11) **EP 4 811 388 A1**
(43) Date of publication of application: **23.09.2026**
(21) Application number: 25165012.3
(22) Date of filing: 20.03.2025
(51) Int. Cl.: G16H 40/63, G16H 50/20

(54) **PREDICTING HEALTH CONDITIONS FROM ORAL CARE DATA**

(71) Applicant: Koninklijke Philips N.V., 5656 AE Eindhoven (NL)
(72) Inventor: VAN DEN DUNGEN, Wilhelmus Andreas Marinus Arnoldus Maria, Eindhoven (NL); KOOIJMAN, Gerben, Eindhoven (NL)
(74) Representative: Philips Intellectual Property & Standards

(57) **Abstract**

A method predicts a health condition based on a gas or gas mixture in an oral cavity by capturing gas sample data during a personal oral care session using a sensor system integrated into a personal oral care device. The captured data is then analyzed to determine a change pattern. A machine-learning model is applied to the change pattern to generate a health condition prediction output, providing a prediction of the health condition.

## Description

### TECHNICAL FIELD

The present disclosure generally relates to the field of electronic personal oral care devices, and more specifically to analyzing gas mixtures present in the oral cavity during oral hygiene routines.

### BACKGROUND

Measuring gases, such as volatile organic compounds (VOCs), present in a subject's breath can provide valuable information related to oral health, and can potentially even indicate early signs of systemic diseases.

For example, US 2018/0303378 A1 discloses predictive disease breath database systems and methods. Breath profiles are captured by a nasal stent, special gum, pacifier, or other device for obtaining volatile/semi volatile compound spectra from samples. A machine-learning model correlates compound spectra with disease indicators.

As another example, JP 2002253584 A discloses a periodontal disease diagnosis device. A gas measuring device with a plurality of semiconductor gas sensors measures a change in the specific resistance value of each semiconductor gas sensor as a time series value by the gas component in the exhausted gas, and a neural network outputs a degree of progression of the periodontal disease of the subject.

Besides such specialized disease diagnosis devices for professional use, gas sensing technologies are also increasingly integrated into personal oral care devices such as electronic toothbrushes. However, this integration faces several challenges. Accurately measuring absolute and specific gas compound/VOC levels remains difficult. Sensors may drift over time and significant sensor-to-sensor variability can hinder the reliability of measurements. Furthermore, focusing on specific VOCs is sometimes challenging due to potential cross-sensitivity between different gases. Slow sensor response times can also limit the practical application of these devices, as accurate results may require extended measurement periods. Integrating advanced sensor systems and complex user instructions into consumer-grade personal care devices poses additional challenges. The inherent variability in gas composition and concentration during oral care routines, coupled with the unique behavior and interactions of each gas, further complicates achieving accurate absolute measurements. Furthermore, differential measurement setups, which aim to improve accuracy by comparing measurements from multiple sensors, require rather complex sensor setups, which limits the practical application of such techniques in personal oral care devices intended for home use.

It is therefore an objective of the present disclosure to provide an improved technique to determine health conditions based on gas mixtures present during personal oral care routines, overcoming the above limitations at least in part.

### SUMMARY OF THE DISCLOSURE

The above and other objectives may be achieved by the subject-matter defined by the independent claims. Advantageous modifications of embodiments of the present disclosure are defined in the dependent claims as well as in the description and the drawings.

One aspect of the present disclosure relates to a method of predicting a health condition. The method may be computer-implemented.

It may be provided that the method comprises obtaining gas sample data. The gas sample data may comprise a plurality of gas samples. The gas sample data may be, or may have been, captured by a sensor system integrated into a personal oral care device. The gas sample data may be, or may have been, captured in an oral cavity of a subject, such as a person, during at least one personal oral care session, such as a toothbrushing session, performed by the subject. The use of a personal oral care device, such as a toothbrush, as a platform for collecting gas sample data has several advantages. For example, the toothbrush head is already in close proximity to the subject's mouth, making it an accurate yet unobtrusive location for gas sampling. Furthermore, the subject typically brushes their teeth twice, maybe three times, a day, providing multiple data points. Moreover, the device is already in use, reducing the need for additional equipment or user interaction.

It may be provided that the method comprises determining a change pattern of the captured gas sample data. It may be provided that the method comprises generating, by a machine-learning model that takes the change pattern as an input, a health condition prediction output. Accordingly, specific health conditions can be predicted by analyzing the unique change patterns of gases or gas mixtures. This may help to detect early signs of diseases and other health conditions, in particular systemic diseases such as diabetes, respiratory diseases, or infections. The inventors have found that absolute gas levels are difficult to measure reliably during a personal oral care session, but change patterns can be observed by relatively inexpensive gas sensors because every gas type, amount and combination will produce its own patterns, which are detectable in the frequency domain. Accordingly, by focusing on change patterns rather than absolute values, a high-quality health condition prediction can be obtained using a comparatively simple, and relatively inexpensive, sensor system. Moreover, focusing on change patterns is more robust against sensor variability and slow drifting over time compared to traditional methods that rely on absolute measurements.

It may be provided that the change pattern used as an input of the machine-learning model comprises a frequency-domain representation of the gas sample data, in particular a spectrogram of the gas sample data. It may be provided that the machine-learning model does not observe any time-domain representation of the gas sample data. Accordingly, this aspect avoids relying solely on time series analysis, which is sensitive to absolute values and drifting sensor readings. Instead, frequency-domain representations are leveraged for more robust pattern recognition. By working in the frequency domain, the method is less sensitive for absolute values, and therefore less dependent on exact absolute gas levels. This results in improved robustness against variations between sensors and slow drift over time, because the use of frequency-domain representations reduces the impact of these issues, as changes in gas mixtures are more important than their absolute values. Furthermore, by analyzing features in the frequency domain (e.g., spectrograms), the machine-learning model can extract relevant information about gas mixture patterns that may not be apparent from time-series data alone.

It may be provided that the sensor system consists of a single gas sensor. Accordingly, complexity and potential points of failure are reduced.

It may be provided that the sensor system comprises a plurality of gas sensors. Accordingly, more complex gas mixtures can be reliably measured.

It may be provided that the method further comprises normalizing the gas sample data before determining the change pattern. By normalizing the gas sample data before determining the change pattern, the absolute values become less critical for the prediction model. This allows the algorithm to focus more on patterns and changes rather than exact measurements.

It may be provided that the machine-learning model further takes as an input environmental data such as temperature, pressure, and/or humidity. It may be provided that the machine-learning model further takes as an input location data. Accordingly, a more feature-rich input is provided to the machine-learning model.

Another aspect of the present disclosure relates to a method of training a machine-learning model for predicting a health condition, in particular for use in a method according to any of the aspects described herein. The method may be computer-implemented. The method may comprise providing a training dataset. The training dataset may comprise a plurality of change patterns of gas sample data. Each change pattern may be annotated with a corresponding health condition. The method may comprise training the machine-learning model using the training dataset.

Another aspect of the present disclosure relates to a data processing apparatus, device or system. The data processing apparatus may comprise means for carrying out a method according to any of the aspects described herein. The data processing apparatus may comprise a memory and one or more processors coupled to the memory, the one or more processors being configured to carry out a method according to any of the aspects described herein.

Another aspect of the present invention relates to a computer program. The computer program may comprise instructions which, when the program is executed by a computer, such as the mentioned data processing apparatus, cause the computer to carry out a method according to any one of the aspects described herein. A computer program may also be referred to as a program, software, a software application, an app, a module, a software module, a script, or code. A computer program may be written in a programming language, including compiled or interpreted languages. A computer program may be deployed in any form, including as a stand-alone product or as a module, component, subroutine, or other unit suitable for use in a computing environment, such as on the mentioned data processing apparatus.

Another aspect of the present disclosure relates to a computer-readable medium having stored thereon a computer program as described herein. Another aspect of the present disclosure relates to a non-transitory computer-readable medium storing a set of instructions that, when executed by one or more processors of an apparatus, cause the apparatus to carry out a method according to any of the aspects described herein.

Another aspect of the present disclosure relates to a machine-learning model trained for use in a method according to any of the aspects described herein.

The terms used herein should generally be construed as understood by the average person skilled in the art, unless explicitly indicated otherwise. The following explanations may guide the understanding:
The term "Artificial intelligence" (AI) may be understood as referring to a branch of computer science that aims to develop machines or software capable of intelligent behavior, typically with the goal to mirror or surpass human intelligence in specific tasks. AI systems are designed to perform complex tasks such as reasoning, learning, perception, problem-solving, and understanding natural language. These systems can typically adapt to new situations and improve their performance over time. The goal of AI is to create systems that can function autonomously and interact with their environment in a human-like manner.

The term "Change pattern" may be understood as an indication of a change in the gas composition or concentration within the oral cavity, which can be used to monitor oral health, detect oral diseases, or analyze the effectiveness of oral care products.

The term "Gas prediction output" may be understood as a data output indicating the predicted composition of gases or gas mixtures present in the oral cavity.

The term "Gas sample data" may be understood as raw data collected from sensors measuring the composition of a gas or gas mixtures present in the oral cavity, such as Volatile Organic Compounds (VOCs), or hydrogen sulfide (H₂S) produced by plaque bacteria, during a subject's personal oral care sessions.

The term "Machine learning" (ML) may be understood as a subset of artificial intelligence that focuses on the development of algorithms and statistical models that enable computers to perform specific tasks without using explicit instructions. Instead, machine-learning systems learn and make predictions or decisions based on data. Machine-learning algorithms build a mathematical model based on sample data, known as training data, to make predictions or decisions without being explicitly programmed to perform the task. Machine learning can be employed in a variety of applications, including image and speech recognition, medical diagnosis, predictive analytics, and many more, where it enables systems to learn from and adapt to new data independently.

The term "Machine-learning algorithm" may be understood as a computational procedure that is designed to analyze data, learn from it, and identify patterns or make decisions based on the input data without being explicitly programmed for the task. Machine-learning algorithms leverage statistical techniques to enable systems to improve their performance on a specific task with more data over time. Machine-learning algorithms are the foundation upon which machine-learning models are built, providing the methods or processes through which data is transformed into actionable insight. Examples of machine-learning algorithms include linear regression, decision trees, support vector machines, and neural networks, among others.

The term "Machine-learning model" may be understood as referring to the output generated when a machine-learning algorithm is trained on a dataset. It represents the knowledge or understanding gained by the algorithm from the data, encapsulating the learned patterns or predictions. Essentially, a machine-learning model is what enables predictions or decisions based on new, unseen data, based on the learning it has derived from the training process. The machine-learning model is typically defined by its parameters, which may be adjusted during the training phase to minimize the difference between the predicted outcome and the actual outcome. Although, strictly speaking, "machine-learning algorithm" and "machine-learning model" have distinct definitions, it is not uncommon for these terms to be used interchangeably in casual discourse. This usage stems from the close relationship between algorithms and models in the workflow of machine-learning projects, where the algorithm is the means of creating the model. Therefore, these terms may be used synonymously herein unless the distinction is decisive.

The term "Neural network" (NN) or "artificial neural network" (ANN) may be understood as a machine-learning or deep-learning model or algorithm. Neural networks are generally inspired by the human brain and typically comprise interconnected nodes or neurons organized into layers. Neural networks can be used to process data and learn from examples, enabling them to perform tasks such as image recognition, natural language processing, and more. A neural network typically comprises an input layer, one or more hidden layers, and an output layer. Through a process called training, neural networks can learn to perform specific tasks by adjusting their internal parameters, or "weights", based on labeled or unlabeled data.

The term "Personal oral care device" may be understood as a device designed for maintaining or improving oral hygiene, with the primary example being an electronic toothbrush. The device may comprise a head portion with cleaning elements (e.g., bristles) configured to clean one or more teeth and oral surfaces of a subject. The device may comprise a body or handle portion for gripping the device. The device may comprise a neck portion extending from the body portion and coupling it to the head portion. The device may comprise an electronic component, such as a motor, configured to articulate the cleaning elements (e.g., rotate, oscillate, or vibrate). The device may comprise a power source, which may be rechargeable batteries housed within the handle. The device. May comprise a control system, including a processor and/or user interface, for operating the device and selecting various modes or functions.

The term "Sensor system" may be understood as a set comprising any number of sensors configured to detect and measure physical parameters, such as gas concentrations in the oral cavity.

The term "Subject" may be understood as a human person who uses the personal oral care device as disclosed herein to maintain or improve their oral hygiene. The subject may be an end-consumer, as opposed to a healthcare professional.

The term "Training" may be understood as referring to the process of teaching a machine-learning model to make predictions or decisions, by exposing it to data for which the outcomes are known. The training process typically involves feeding a training dataset into a machine-learning algorithm, which then uses statistical analysis to learn the patterns or relationships within the data. During training, the algorithm iteratively adjusts the parameters of the model to minimize the difference between the predicted outcomes and the actual outcomes in the training data. This adjustment process is typically guided by a loss function, which measures the accuracy of the model's predictions. The goal of training is to produce a model that accurately represents the underlying structure of the data, enabling it to make reliable predictions about new, unseen data. Supervised learning involves training a model on a labeled dataset, where each example in the training data is paired with the correct output. The model learns to predict the output from the input data. Unsupervised learning involves training a model on data without labeled responses. The model tries to find patterns and relationships in the data on its own. Semi-supervised learning combines both labeled and unlabeled data during the training process, which can be beneficial when acquiring a fully labeled dataset is costly or impractical.

These and other aspects of the invention will be apparent from and elucidated with reference to the embodiment(s) described hereinafter.

### BRIEF DESCRIPTION OF THE DRAWINGS

The present disclosure may be better understood by reference to the following drawings:
FIG. 1 illustrates a schematic view of a process for predicting a health condition in accordance with one embodiment.
FIG. 2 illustrates exemplary gas measurement patterns for different gases in accordance with one embodiment.
FIG. 3 illustrates two exemplary time series signals and corresponding spectrograms in accordance with one embodiment.
FIG. 4 illustrates an exemplary architecture of a machine-learning model in accordance with one embodiment.
FIG. 5 illustrates another exemplary architecture of a machine-learning model in accordance with one embodiment.
FIG. 6 illustrates an exemplary baseline training process in accordance with one embodiment.
FIG. 7 illustrates an exemplary personalization and federated learning technique in accordance with one embodiment.
FIG. 8 illustrates an exemplary hardware implementation in accordance with one embodiment.
FIG. 9 illustrates a method of predicting a gas mixture in accordance with one embodiment.
FIG. 10 illustrates a method of training a machine-learning model for predicting a gas mixture in accordance with one embodiment.

### DETAILED DESCRIPTION

In the following, representative embodiments illustrated in the accompanying drawings will be explained. It should be understood that the illustrated embodiments and the following descriptions refer to examples which are not intended to limit the embodiments to one preferred embodiment.

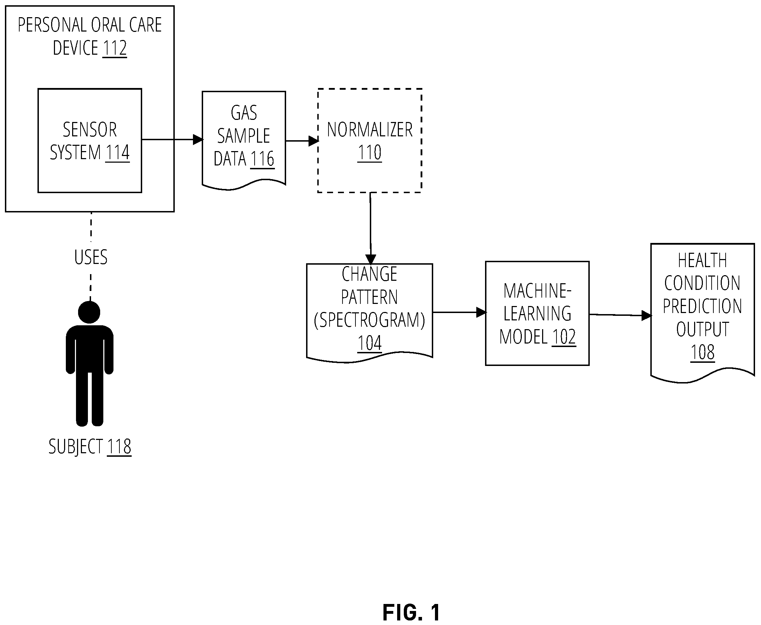

FIG. 1 illustrates a schematic view of a process for predicting a health condition in accordance with certain embodiments. A subject 118 uses a personal oral care device 112 during a personal oral care session. In the scenario described here by way of example, the personal oral care device 112 is an electronic toothbrush and the personal oral care session is a toothbrushing session which typically takes about two minutes. The personal oral care device 112 comprises a sensor system 114 which captures gas sample data 116 in an oral cavity of the subject 118 during the personal oral care session. The gas sample data 116 comprises a plurality of gas samples. The captured gas sample data 116 may comprise data, i.e., gas samples, from one or multiple personal oral care sessions performed by the subject 118. The captured gas sample data 116 is fed into an optional normalizer 110. A change pattern 104 of the (normalized) gas sample data 116 is determined, e.g., a spectrogram. The change pattern 104 serves as an input into a machine-learning model 102 which generates a health condition prediction output 108.

Accordingly, the machine-learning model 102 serves as a health condition prediction model which takes the entire block of gas samples (collected during one or multiple personal oral care sessions) and predicts the health condition prediction output 108.

In certain embodiments, when a single health condition is considered, the health condition prediction output 108 comprises a single value that is representative of the actual health condition. This way, the machine-learning model 102 may be understood as a multidimensional classifier with a multidimensional input (the gas sample data 116) and a one-dimensional output (the health condition prediction output 108).

In other embodiments, when multiple health conditions are considered, the health condition prediction output 108 comprises multiple values. This way, the machine-learning model 102 may be understood as a multidimensional classifier with a multidimensional input (the gas sample data 116) and a multidimensional output (the health condition prediction output 108).

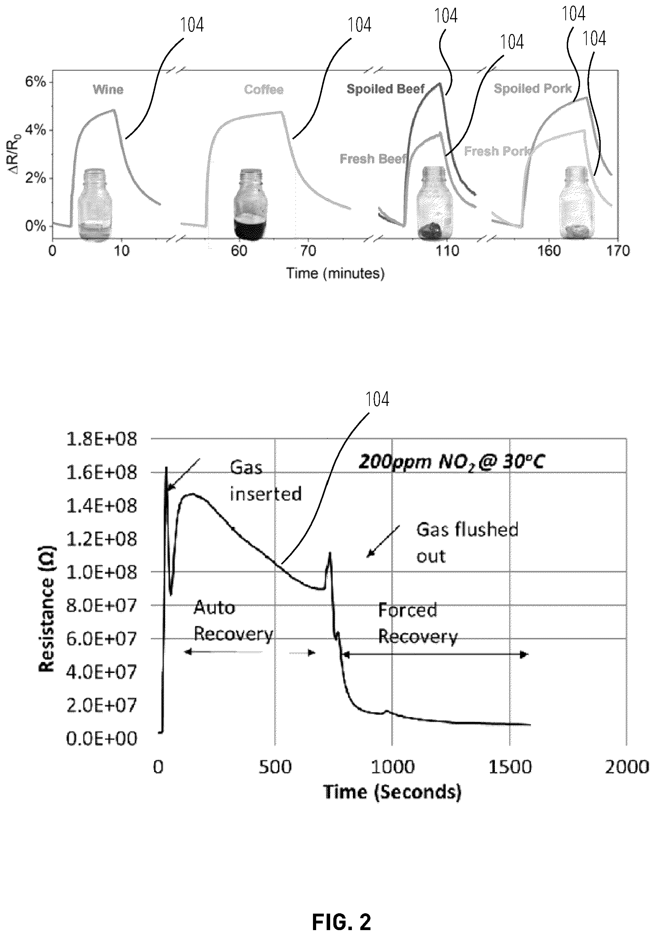

FIG. 2 illustrates exemplary gas measurement patterns, i.e., change patterns 104, for different gases in accordance with certain embodiments. In the upper part of FIG. 2, the example illustrates change patterns 104 of gases produced by wine, coffee, beef (fresh and spoiled) and pork (fresh and spoiled). The change patterns 104 are plotted as the relative resistance (*ΔR*/*R*₀) as a function of time.

A dynamic environment such as an oral cavity typically results in more complex change patterns 104, an example of which is shown in the lower part of FIG. 2. The illustrated graph is from a gas mix experiment in a measurement chamber. The auto recovery is the response of the gas sensor on the gas mixture, the forced recovery is the removal of the gas mixture and the response of the gas sensor. This graph serves to illustrate that gas mixtures in combination with gas sensor can have a dynamic behavior that can be used to identify the gas mixture applied or detected.

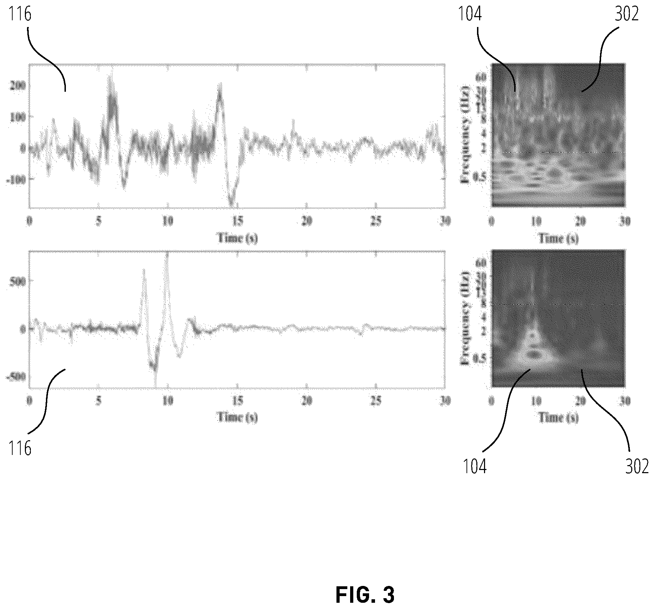

FIG. 3 illustrates two exemplary time series signals and corresponding spectrograms 302 in accordance with certain embodiments. The time series signals represent captured gas sample data 116, and the spectrograms 302 represent their change patterns 104. In certain embodiments, the change patterns 104 provided as an input to the machine-learning model 102 are in the form of spectrograms 302, as one example of a frequency-domain representation. In other words, these embodiments are looking into the frequency (change) domain over time, making the prediction less dependent on absolute values and less sensitive for drifting sensor readings.

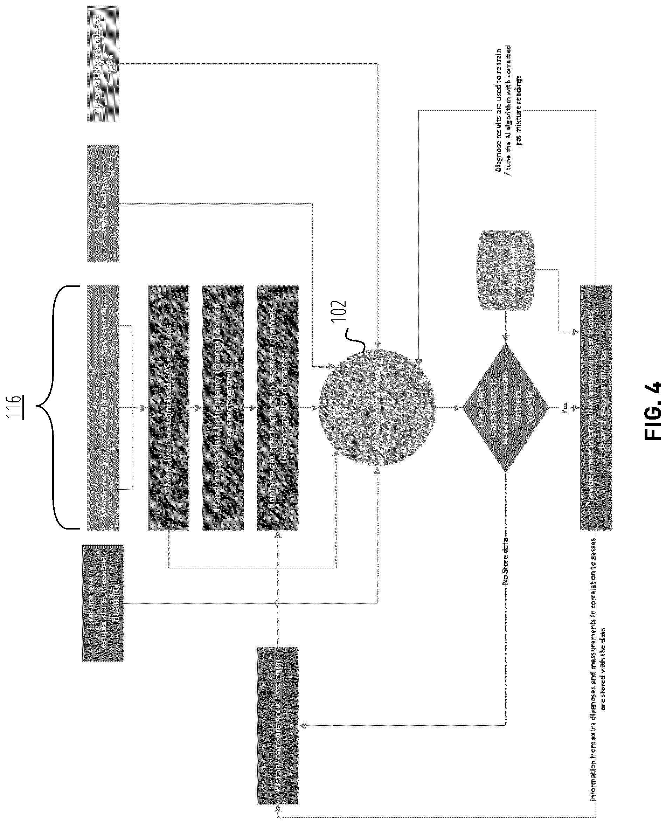

FIG. 4 illustrates an exemplary architecture of a machine-learning model 102 in accordance with certain embodiments. The input data comprises gas sample data 116 specific to predetermined sensor profiles (selected based on relevance to targeted gas mixtures and, optionally, health prediction). Additionally, environmental data, including temperature, pressure, and/or humidity, can influence gas pattern readings and form an independent source of information. Furthermore, location data and/or personal health data can be utilized as an input, as the change patterns exhibited by the gas mixture can vary significantly depending on the location and subject's personal care routine. The gas sample data 116 is normalized by combining gas readings, following preprocessing, to enable the detection of overlapping information across different gas spectrograms in various channels.

In one embodiment, the machine-learning model 102 comprises, or is, a convolutional neural network (CNN), fully connected network, or a combination thereof. An advantage of using a CNN as the machine-learning model in this context is its ability to analyze image-like data, such as spectrograms. Spectrograms comprise two-dimensional representations of gas sample data change patterns over time, which can be thought of as "images" with temporal information embedded within them. CNNs are particularly well-suited for analyzing these types of images.

In another embodiment, the machine-learning model 102 comprises, or is, a long short-term memory (LSTM)-based machine-learning model. An advantage of using an LSTM as the machine-learning model in this context is its ability to leverage historical data and data change patterns (spectrograms). LSTMs are particularly well-suited for analyzing sequential or time-series data. The LSTM can learn from historical data to understand how different gases respond differently over various temperature profiles. By processing spectrograms (time-frequency frequency-domain representations), the LSTM can identify changes in gas sample data change patterns over time. Note that this embodiment still does not look at time series data of a sensor, but rather at spectrograms in time in the frequency domain. This could be conceptualized similar to a generating a movie from all the spectrogram images in sequence, which will make changes in the spectrograms over time visible to the model.

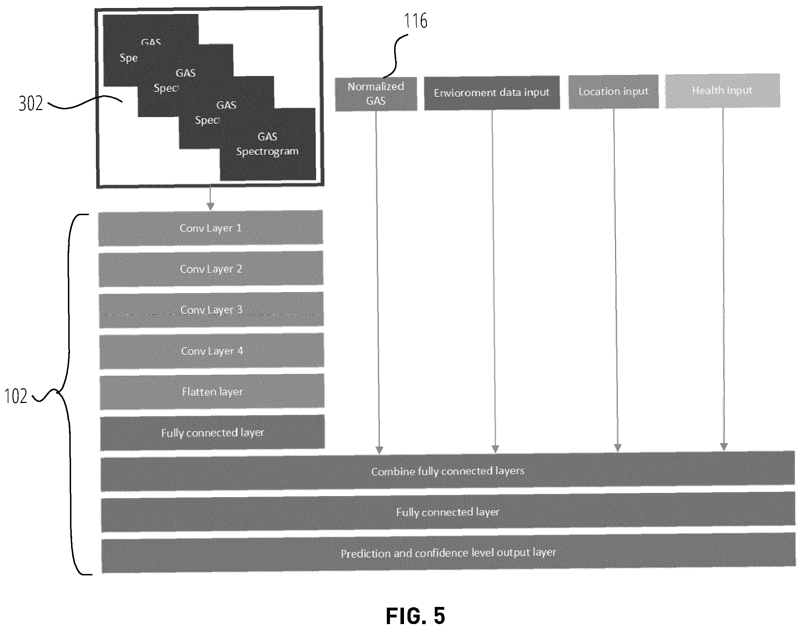

FIG. 5 illustrates another exemplary architecture of a machine-learning model 102 in accordance with certain embodiments. The embodiment described is a combination of a convolutional neural network (CNN) and fully connected layers. In the illustrated embodiment, the CNN comprises a plurality of convolutional layers (four layers in FIG. 5), a flattening layer, and a fully connected layer. Gas spectrograms 302 are input into the CNN. In addition, normalized gas sample data 116 and, optionally, additional inputs (environmental data, location data and/or health data) are combined with the flattened output of the CNN and additional layers to form the machine-learning model 102.

In certain embodiments, the CNN is trained to recognize change patterns 104 and features in the spectrogram data, which are then represented in the fully connected layers and combined with the additional data to generate a health condition prediction output 108 indicative of health problems. In addition, the machine-learning model 102 may output a confidence level for relating the health condition prediction output 108 to a health problem or condition.

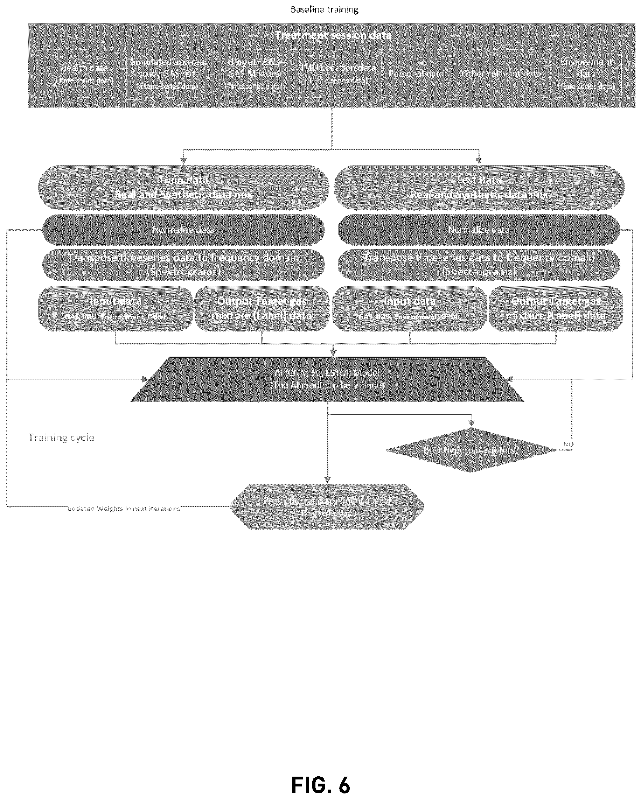

FIG. 6 illustrates an exemplary baseline training process in accordance with certain embodiments. The input data comprises time-series data (real and/or synthetic), environmental data, and location data, which can all be converted to spectrograms, represented in the frequency domain over time. If needed, other relevant data, such as personal data, can be more static and fed into the algorithm further down into the fully connected layers, as shown in FIG. 5. (note that in FIG. 5, the location and health inputs are also considered more static and connected further down the model, as an alternative embodiment).

For the embodiment shown in FIG. 5, the machine-learning model 102 is trained on gas sample data 116, i.e., real measured gases and target real mixtures present in the dataset. The measured gases are representative of those measured by sensors in practice, while the target gas mixtures are the theoretical mixtures related to health issues. Both the real measured and target mixtures can be real and/or lab model-generated and/or simulated synthetic data. When limiting the machine-learning model 102 to gas mixture prediction and not health prediction, the training data can be more effectively generated and proven via models, thereby removing the complex human system from the model.

In certain embodiments, the real and synthetic data are mixed to ensure that the data covers as many real-life situations as possible. Normalization and conversion to frequency-based data (spectrograms) are preferably performed after normalization. The data pairs are then provided to the machine-learning model 102, with inputs including gas, environment, and/or location information, and output labels including target gas mixture information related to the real measured gases, time slot, location, and/or personal data.

In certain embodiments, the machine-learning model 102 is iteratively trained, and weights are updated. If necessary, hyperparameters can be tuned to optimize performance and accuracy. The output of the machine-learning model 102 is a gas mixture prediction and, optionally, a confidence level. This prediction may be correlated to a known related health status and/or condition in a subsequent step behind the machine-learning model 102.

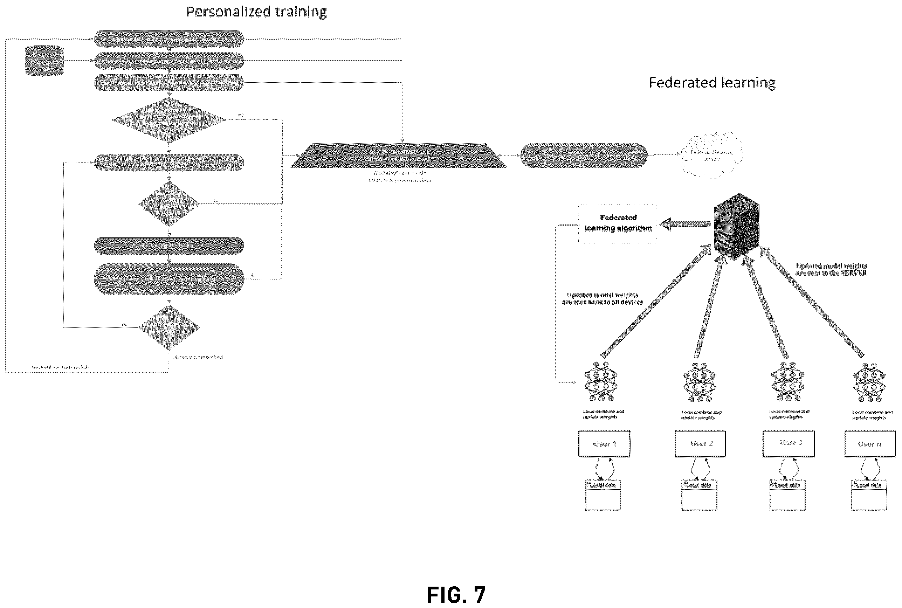

FIG. 7 illustrates an exemplary personalization and federated learning technique in accordance with certain embodiments, which is particularly usable in a disease indication prediction. When health data is available, such as health watch readings, user feedback and/or doctor feedback, such data can be used to verify the accuracy of generated disease indications, and to update the machine-learning model 102 as necessary.

In an embodiment where health predictions are made based on predicted gas mixtures, the subject can be informed if the prediction deviates significantly from expected gas mixtures related to the provided health information. Furthermore, when a potential safety risk is identified based on incorrect predictions, preventive actions and/or warnings can be provided to the user.

In certain embodiments, the machine-learning model 102 can be updated to improve accuracy and prevent future errors, based on user feedback and the severity of the deviation, through a iterative process of retraining and fine-tuning. Notably, the gas prediction outputs remain predictions, and the disease indication predictions are likewise predictions, serving as an aid for an actual diagnosis, rather than a diagnosis in themselves.

In certain embodiments, weights and/or weight deltas between different subjects may be shared, and used to update and retrain the machine-learning model 102. This way, more reliable and accurate predictions can be made, while minimizing privacy concerns through the use of general gas mixture predictions. The weights are related the general gas mixture predictions, not specific personal health predictions, so they are much less sensitive for privacy data sharing challenges in federated learning.

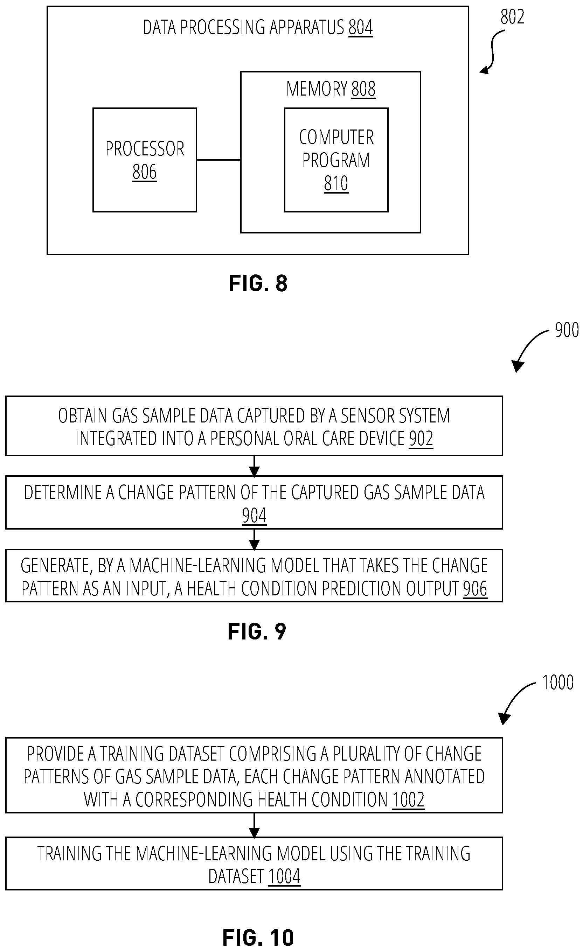

FIG. 8 illustrates a schematic block diagram of computer hardware usable for carrying out one or more aspects disclosed herein. As can be seen, a system 802 comprises a data processing apparatus 804. The data processing apparatus 804 comprises one or more processors, one of which is exemplarily shown as processor 806. The data processing apparatus 804 comprises a memory 808. The one or more processors 806 are communicatively coupled to the memory 808. The memory 808 comprises a computer program 810. The computer program 810 may implement some or all aspects of the disclosed methods and functionalities.

FIG. 9 illustrates a method 900 of predicting a health condition in accordance with one embodiment. In step 902, method 900 obtains gas sample data 116 captured by a sensor system 114 integrated into a personal oral care device 112, the gas sample data 116 being captured in an oral cavity of a subject 118 during at least one personal oral care session performed by the subject 118. In step 904, method 900 determines a change pattern 104 of the captured gas sample data 116. In step 906, method 900 generates, by a machine-learning model 102 that takes the change pattern 104 as an input, a health condition prediction output 108.

FIG. 10 illustrates a method of training a machine-learning model 102 for predicting a health condition in accordance with one embodiment. In step 1002, method 1000 provides a training dataset comprising a plurality of change patterns 104 of gas sample data 116, each change pattern 104 annotated with a corresponding health condition. In step 1004, method 1000 trains the machine-learning model 102 using the training dataset.

In summary, certain embodiments have been illustrated which involve an AI prediction model based on gas change patterns (during personal care routines twice a day every day). Time series data of single sessions, per day or even over multiple sessions, and or a moving window approach can be used as input for the prediction models. The gas change patterns comprise gas measurements with sensors that are selected to be in the range of gases or mixtures that are known to be related to the to be predicted health issues. The requirements for the sensors and profiles are limited. Furthermore, absolute values are less important and not the single source of information, also slow drifting over time as well as variation between sensors are not the most critical aspects when looking at change patterns.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Embodiments of the present disclosure may be implemented on a computer system. The computer system may be a local computer device (e.g., personal computer, laptop, tablet computer or mobile phone) with one or more Processors and one or more storage devices or may be a distributed computer system (e.g., a cloud computing system with one or more Processors and one or more storage devices distributed at various locations, for example, at a local client and/or one or more remote server farms and/or data centers). The computer system may comprise any circuit or combination of circuits. In one embodiment, the computer system may include one or more Processors which can be of any type. As used herein, Processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics Processor, a digital signal Processor (DSP), multiple core Processor, a field programmable gate array (FPGA), or any other type of Processor or processing circuit. Other types of circuits that may be included in the computer system may be a custom circuit, an application-specific integrated circuit (ASlC), or the like, such as, for example, one or more circuits (such as a communication circuit) for use in wireless devices like mobile telephones, tablet computers, laptop computers, two-way radios, and similar electronic systems. The computer system may include one or more storage devices, which may include one or more Memory elements suitable to the particular application, such as a main Memory in the form of random access Memory (RAM), one or more hard drives, and/or one or more drives that handle removable media such as compact disks (CD), flash Memory cards, digital video disk (DVD), and the like. The computer system may also include a display device, one or more speakers, and a keyboard and/or controller, which can include a mouse, trackball, touch screen, voice-recognition device, or any other device that permits a system user to input information into and receive information from the computer system.

Some or all of the method steps may be executed by (or using) a hardware apparatus, like for example, a Processor, a microprocessor, a programmable computer or an electronic circuit. In some embodiments, some one or more of the most important method steps may be executed by such an apparatus.

Depending on certain implementation requirements, embodiments of the present disclosure can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH Memory, having electronically readable control signals stored thereon, which cooperate (or are capable of cooperating) with a programmable computer system such that the respective method is performed. Therefore, the digital storage medium may be computer readable.

Some embodiments comprise a data carrier having electronically readable control signals, which are capable of cooperating with a programmable computer system, such that one of the methods described herein is performed.

Generally, embodiments of the present disclosure can be implemented as a computer program product with a program code, the program code being operative for performing one of the methods when the computer program product runs on a computer. The program code may, for example, be stored on a machine-readable carrier.

Other embodiments comprise a computer program for performing one of the methods described herein, stored on a machine-readable carrier.

A further embodiment is a computer program having a program code for performing one of the methods described herein, when the computer program runs on a computer.

A further embodiment is a storage medium (or a data carrier, or a computer-readable medium) comprising, stored thereon, the computer program for performing one of the methods described herein when it is performed by a Processor. The data carrier, the digital storage medium or the recorded medium are typically tangible and/or non-transitory.

A further embodiment is an apparatus as described herein comprising a Processor and the storage medium.

A further embodiment is a data stream or a sequence of signals representing the computer program for performing one of the methods described herein. The data stream or the sequence of signals may, for example, be configured to be transferred via a data communication connection, for example, via the internet.

A further embodiment is a processing means, for example, a computer or a programmable logic device, configured to, or adapted to, perform one of the methods described herein.

A further embodiment is a computer having installed thereon the computer program for performing one of the methods described herein.

A further embodiment is an apparatus or a system configured to transfer (e.g., electronically or optically) a Computer program for performing one of the methods described herein to a receiver. The receiver may, for example, comprise a computer, a mobile device, a Memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

### REFERENCE SIGNS:

102 machine-learning model
104 change pattern
106 gas prediction output
108 health condition prediction output
110 normalizer
112 personal oral care device
114 sensor system
116 gas sample data
118 subject
302 spectrogram
802 system
804 data processing apparatus
806 processor
808 memory
810 computer program
900 method
902 step
904 step
906 step
1000 method
1002 step
1004 step

## Claims

1. A method of predicting a health condition, comprising:
obtaining gas sample data (116) captured by a sensor system integrated into a personal oral care device, the gas sample data being captured in an oral cavity of a subject during at least one personal oral care session performed by the subject;
determining a change pattern (104) of the captured gas sample data; and
generating, by a machine-learning model (102) that takes the change pattern (104) as an input, a health condition prediction output (108).

2. The method of claim 1, wherein the change pattern (104) used as an input of the machine-learning model comprises a frequency-domain representation of the gas sample data.

3. The method of claim 2, wherein the frequency-domain representation comprises a spectrogram (302) of the gas sample data.

4. The method of any one of claims 1 to 3, wherein the sensor system consists of a single gas sensor.

5. The method of any one of claims 1 to 3, wherein the sensor system comprises a plurality of gas sensors.

6. The method of any one of claims 1 to 5, further comprising:
normalizing the gas sample data before determining the change pattern.

7. The method of any one of claims 1 to 6, wherein the machine-learning model further takes as an input environmental data such as temperature, pressure, and/or humidity.

8. The method of any one of claims 1 to 7, wherein the machine-learning model further takes as an input location data.

9. A method of training a machine-learning model (102) for predicting a health condition, in particular for use in the method of any one of claims 1 to 8, comprising:
providing a training dataset comprising a plurality of change patterns (104) of gas sample data (116), each change pattern annotated with a corresponding health condition; and
training the machine-learning model (102) using the training dataset.

10. A data processing apparatus comprising means for carrying out the method of any one of claims 1 to 9.

11. A computer program or a computer-readable medium storing a computer program, the computer program comprising instructions which, when the program is executed on a computer, cause the computer to carry out the method of any one of claims 1 to 9.

12. A machine-learning model (102) trained for use in the method of any one of claims 1 to 8.
